(19) Europäisches Patentamt / European Patent Office / Office européen des brevets

(11) **EP 4 043 568 A1**

(12) **EUROPEAN PATENT APPLICATION**
published in accordance with Art. 153(4) EPC

(43) Date of publication:
**17.08.2022 Bulletin 2022/33**

(21) Application number: **20873684.3**

(22) Date of filing: **07.10.2020**

(51) International Patent Classification (IPC):
**C12N 15/12** (2006.01)   **G01N 33/15** (2006.01)
**A01K 67/027** (2006.01)

(52) Cooperative Patent Classification (CPC):
**A01K 67/027; G01N 33/15**

(86) International application number:
**PCT/JP2020/038004**

(87) International publication number:
**WO 2021/070866 (15.04.2021 Gazette 2021/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.10.2019 JP 2019185245**

(71) Applicant: **AlzMed, Inc.**
**Tokyo 113-8485 (JP)**

(72) Inventors:
• **SHIRAO, Tomoaki**
  **Maebashi-shi, Gunma 371-8510 (JP)**

• **SEKINO, Yuko**
  **Maebashi-shi, Gunma 371-8510 (JP)**
• **HANAMURA, Kenji**
  **Maebashi-shi, Gunma 371-8510 (JP)**
• **YAMAZAKI, Hiroyuki**
  **Maebashi-shi, Gunma 371-8510 (JP)**
• **KOGANEZAWA, Noriko**
  **Maebashi-shi, Gunma 371-8510 (JP)**

(74) Representative: **Engelhard, Markus**
**Boehmert & Boehmert**
**Anwaltspartnerschaft mbB**
**Pettenkoferstrasse 22**
**80336 München (DE)**

(54) **NON-HUMAN ALZHEIMER'S DISEASE MODEL ANIMAL AND METHOD FOR PRODUCING SAME**

(57)    The current Alzheimer's disease model mouse, developed by manipulating the expression of a gene directly related to distinctive pathology that is specific to the disease, such as acceleration of amyloid plaque deposition, is a model of a preclinical state, or of a pathology that is fundamentally different from human Alzheimer's disease. In view of the current state described above, the present invention addresses the problem of developing a non-human transgenic animal in which Alzheimer's pathology is more accurately reflected, and thereby elucidating a disease mechanism or contributing to drug discovery. In the present invention, by heterozygous knockout of the drebrin gene of a non-human Alzheimer's disease model animal used as a base, senescence risk can be imparted to the conventional non-human Alzheimer's disease model animal. Alzheimer's pathology is more accurately reflected in this non-human Alzheimer's disease model animal.

FIG.5

## Description

[Technical Field]

The present invention relates to a genetically recombinant non-human animal that reflects a pathological condition of Alzheimer's disease more accurately compared to conventional non-human animal Alzheimer's disease model by halving the drebrin expression level, a manufacturing method of the genetically recombinant non-human animal, and a method of screening an agent for treating and/or preventing Alzheimer's disease using the genetically recombinant non-human animal.

[Background Art]

The increase in patients of Alzheimer's disease, which is the primary cause of dementia, has become a major societal concern in recent years. Early establishment of a therapeutic drug or therapeutic method thereof is desired. Therapeutic drugs and therapeutic methods for a specific disease are generally investigated through analysis using an animal model of the disease.

Senile plaques are observed in the postmortem brain of Alzheimer's disease patients, which are known as aggregations of "amyloid β protein" (amyloid plaques). Deposition of amyloid β proteins is the earliest lesion that can be pathologically confirmed. It is reported that amyloid β proteins aggregate and directly exhibit neurotoxicity. Currently, it is widely accepted that abnormal amyloid β protein production and accumulation is broadly associated with the development of Alzheimer's disease in view of genetic analysis of familial Alzheimer's disease patients. This is known as the amyloid cascade hypothesis. In this manner, it is widely accepted that amyloid β proteins are the primary cause of Alzheimer's disease in view of numerous studies (Non Patent Literature 1).

It is understood that senescence is the strongest risk factor for Alzheimer's disease (Non Patent Literature 2). However, the primary cause thereof is amyloid β proteins as described above. Thus, mouse models have been developed in the past by manipulating the expression of a gene that is directly associated with a significant pathological condition which is specific to a disease such as promotion of amyloid plaque deposition (Non Patent Literature 1). However, promotion of amyloid plaque deposition may face challenges in inducing dementia, which is a clinical symptom, or neurofibrillary tangle, which is the histopathology of late stage Alzheimer's disease, and may rather induce a significant pathological change. Thus, it is understood that existing mouse Alzheimer's models are preclinical models or mouse models with a pathological condition that is fundamentally different from human Alzheimer's disease.

The inventors were the first in the world to discover the actin-binding protein drebrin, which is expressed in neurons during the developmental process at a large quantity (see, for example, Non Patent Literatures 3 and 4). The inventors have already proven that: drebrin is associated with morphogenesis, especially neurite formation, in neurons by changing the attribute of actin fibers (see, for example, Non Patent Literatures 5 to 7); and drebrin is present throughout the cell body and neurite in migrating neurons during development, but is present specifically in the spinal structure in mature neurons (see, for example, Non Patent Literatures 8 to 10). There are two isoforms of drebrin, i.e., embryonic type drebrin E and adult type drebrin A (see, for example, Non Patent Literature 4). Drebrin A found specifically in the dendritic spine of mature neurons has a characteristic of being expressed in only neurons (see, for example, Non Patent Literatures 9 and 10).

The inventors have also reported that drebrin in dendritic spines disappeared in an extended range in dementia such as Alzheimer's disease (Non Patent Literature 11). The inventors have recently created drebrin complete knockout mice (DXKO) and isoform converting mechanism deficient mice A (DAKO) to elucidate the role of drebrin in synaptic plasticity. When the effect on the learning behavior and synaptic plasticity was studied, a notable morphological abnormality in the brain was not found in DAKO mice, but fear conditioned memory disorder and impairment in the hippocampal synaptic plasticity were observed (Non Patent Literature 12). Abnormalities in neuron migration and olfactory sense are also suggested in DXKO homozygous mice (Non Patent Literature 13).

[Citation List]

[Non Patent Literature]

[NPL 1] Takashi Saito, Takaomi Nishimichi "Arutsuhaimabyo no Moderu Mausu [mouse model for Alzheimer's disease]" (Folia Pharmacol. Jpn.) 144, 250-252, 2014
[NPL 2] Aging Cell. 17, e12802, 2018
[NPL 3] J. Neurochem. 44, 1210-1216, 1985

[NPL 4] J. Biochem. 117, 231-236, 1995
[NPL 5] J. Neurosci. Res. 38, 149-159, 1994
[NPL 6] Exp. Cell Res. 215, 145-153, 1994
[NPL 7] J. Biol. Chem. 269, 29928-29933, 1994
[NPL 8] J. Neurosci. 15, 7161-7170, 1996
[NPL 9] Dev. Brain Res. 29, 233-244, 1986
[NPL 10] Brain Res. 413, 374-378, 1987
[NPL 11] J Neurosci. Res. 43(1), 87-92, 1996
[NPL 12] Neuroscience. 165(1), 138-50, 2010
[NPL 13] Eur. J. Neuroscience, 46, 2214-2228, 2017

[Summary of Invention]

[Technical Problem]

As described above, it is understood that existing mouse Alzheimer's disease models developed by manipulating the expression of a gene that is directly associated with a significant pathological condition which is specific to a disease such as promotion of amyloid plaque deposition are preclinical models or mouse models with a pathological condition that is fundamentally different from human Alzheimer's disease. The present invention was invented in view of such a circumstance in order to contribute to elucidation of the disease mechanism or research for drug development by developing a genetically recombinant non-human animal that reflects the pathological condition of Alzheimer's disease more accurately.

[Solution to Problem]

The inventors studied conventional drebrin knockout mice (DXKO) to discover that complete knockout of drebrin results in an abnormality in synaptic plasticity associated with memory and learning, but there is no significant change in the expression level of various synaptic functional proteins other than drebrin (Non Patent Literature 13), and a significant change was not detected in terms of behavioral abnormalities other than olfactory sense related behaviors. The inventors conducted diligent studies while focusing on the fact that the drebrin level in the brain gradually decreases in humans from about 20 years old and is nearly halved at 60 years old (see J Neuropathol Exp Neurol. 1999 Jun; 58(6): 637-43) to arrive at the notion that a phenotype reflecting senescence, which is different from complete knockout of drebrin, may be exhibited when drebrin is halved as seen in elderlies who are 60 years old or older. In this regard, when animals with heterozygous knockout of a drebrin gene were created and analyzed, abnormalities in synapses and behavioral abnormalities were unexpectedly observed, which were not observed in DXKO mice with the drebrin genes completely knocked out. In this regard, the inventors have conceived the notion that an animal model having the strongest risk factor of Alzheimer's disease, i.e., senescence, may be able to be created by halving the amount of drebrin in a currently used mouse Alzheimer's disease model, and prepared mice (5xFAD/DXKO$^{+/-}$) heterozygously having a 5 x FAD gene and drebrin gene (+/-) by crossbreeding a 5xFAD mouse (mouse having five familial Alzheimer's disease genes) with a drebrin knockout mouse (homo). It was found from comparing the amount of amyloid β and the amount of PSD-95 protein in the resulting mice that amyloid β accumulation is promoted, whereas PSD-95 is significantly reduced in the crossbred mice relative to 5xFAD mice. The present invention was completed from these findings.

Specifically, the present invention relates to the following.

[1] A genetically recombinant non-human animal characterized by heterozygous knockout of a drebrin gene of a base non-human animal Alzheimer's disease model.
[2] The genetically recombinant non-human animal of [1], characterized by being a drebrin A and drebrin E heterozygous knockout form.
[3] The genetically recombinant non-human animal of [1] or [2], characterized in that the base non-human animal Alzheimer's disease model is a non-human animal Alzheimer's disease model with accumulation of amyloid β in a brain.
[4] The genetically recombinant non-human animal of any one of [1] to [3], characterized in that the base non-human animal Alzheimer's disease model is a mouse Alzheimer's disease model.
[5] The genetically recombinant non-human animal of [4], characterized in that the mouse Alzheimer's disease model is a 5 x FAD mouse.
[6] A method of preparing a genetically recombinant non-human animal that develops Alzheimer's disease, characterized in that a base non-human animal Alzheimer's disease model is crossbred with a drebrin gene knockout non-human animal.

[7] A method of screening an agent for treating and/or preventing Alzheimer's disease, comprising the following steps (a) and (b):

(a) administering a test substance to the genetically recombinant non-human animal of any one of [1] to [5]; and
(b) evaluating a therapeutic and/or prophylactic effect on Alzheimer's disease.

[Advantageous Effects of Invention]

The present invention can readily achieve synaptic vulnerability due to senescence, which is difficult to achieve in short-lived animal models such as mice, by halving drebrin. A mouse model of a disease with a risk of senescence can be created from a mouse model of a disease that requires senescence by crossbreeding a conventional mouse model and a drebrin knockout mouse to produce a drebrin heterozygous knockout mouse.

[Brief Description of Drawings]

[Figure 1] Figure **1** is (A) a schematic diagram showing the method of creating drebrin knockout (DXKO) mice in Example 1 and (B) a diagram showing results of genotyping for sorting DXKO homozygous mice from DXKO heterozygous mice in Example 1.
[Figure 2] Figure **2** is a diagram showing results of analyzing the drebrin expression levels of wild-type (WT), DXKO heterozygous mouse (DXKO$^{+/-}$), and DXKO homozygous mouse (DXKO$^{-/-}$) by Western blotting in Example 2.
[Figure 3] Figure **3** is a diagram showing results of measuring the viable neuron count, dendrite length, and drebrin aggregation image count (total drebrin cluster count and drebrin cluster density) of wild-type (WT), DXKO heterozygous mouse (Het), and DXKO homozygous mouse (KO) in Example 3.
[Figure 4] Figure **4** is a diagram showing results of analyzing NMDA receptor activity of wild-type and DXKO heterozygous mouse (hetero-type) in Example 4.
[Figure 5] Figure **5** is a diagram showing results of a rotarod test on wild-type (WT), DXKO heterozygous mouse (Het), and DXKO homozygous mouse (KO) in Example 6.
[Figure 6] Figure **6** is a diagram showing (A) the Y-maze used in Example 7 and (B) results of analyzing the behavioral change in wild-type (WT), DXKO heterozygous mouse (hetero-type), and DXKO homozygous mouse (homo-type) in Example 7. A decrease in locomotion was evaluated by a decrease in exploratory behavior, and a decrease in spatial working memory was evaluated by a decrease in turn alternation.
[Figure 7] Figure **7** is a diagram showing results of comparing amyloid β accumulation in the brain of a 5xFAD/DXKO$^{+/-}$ mouse and a 5xFAD mouse by Western blotting in Example 9.
[Figure 8] Figure **8** is a diagram showing results of comparing PSD95 expression in the brain of a 5xFAD/DXKO$^{+/-}$ mouse and a 5xFAD mouse by Western blotting in Example 9.

[Description of Embodiments]

The present invention relates to a genetically recombinant non-human animal characterized by heterozygous knockout of a drebrin gene of a base non-human animal Alzheimer's disease model (hereinafter, referred to as the "genetically recombinant non-human animal of the invention"). In this regard, drebrin has two primary isoforms (embryonic type isoform drebrin E and adult type isoform drebrin A), which are actin-binding proteins produced by selective splicing from a single drebrin gene. The difference between drebrin A and drebrin E is the presence/absence of an Ins2 sequence (46 amino acid residues). An Ins2 sequence is inserted into the molecule in drebrin A.
As used herein, "heterozygous knockout of a drebrin gene of a base non-human animal Alzheimer's disease model" means that one of the drebrin genes on a homologous chromosome of a non-human animal Alzheimer's disease model used as a base is knocked out. Since one of the drebrin genes on a homologous chromosome is knocked out in the genetically recombinant non-human animal of the invention, as a result, expression of drebrin is not completely lost, but is lower than that of the base non-human animal Alzheimer's disease model. The genetically recombinant non-human animal of the invention is preferably an animal produced from heterozygous knockout of both drebrin A and drebrin E of a base non-human animal Alzheimer's disease model, which can reflect a pathological condition of Alzheimer's disease more accurately, such as elevated amyloid β accumulation in the brain relative to the base non-human animal Alzheimer's disease model. While the reason why the genetically recombinant non-human animal of the invention reflects a pathological condition of Alzheimer's disease more accurately relative to the base non-human animal Alzheimer's disease model is not clear, it is understood that this is because halving the amount of drebrin can provide the strongest risk factor for Alzheimer's disease, i.e., senescence. Therefore, the genetically recombinant non-human animal of the invention can be any animal with an expression level of drebrin that is lower than that of a base non-human animal Alzheimer's disease model. The expression level of drebrin may also be reduced using a known method of adjusting gene expression levels such as gene knock down instead of, or together with, heterozygous knockout.

Examples of the base non-human animal Alzheimer's disease model in the present invention include non-human animal Alzheimer's disease models with accumulation of amyloid β such as non-human animal models overexpressing a precursor protein of amyloid β (amyloid precursor protein: APP) and non-human animal models introduced with a familial mutation of Alzheimer's disease in an APP gene. In this regard, examples of the non-human animals include mice, rats, rabbits, guinea pigs, hamsters, and the like. In particular, rodents such as mice are preferable. As the base non-human animal Alzheimer's disease model, a non-human animal Alzheimer's disease model described in, for example, the database on the ALZFORUM website (https://www.alzforum.org/databases) can be used. Specific examples include mouse models such as Tg2576, APP23, PDAPP, J20, APPPS1, APP/PS1ΔE9, TgCRND8, 3xTg, 5xFAD, APP/PS1-dKI, App$^{NL-F}$-KI, and App$^{NL-G-F}$-KI, but are not limited to the non-human animal Alzheimer's disease models described in said database. In one embodiment, the base non-human animal Alzheimer's disease model does not comprise drebrin A and/or drebrin E knockout form, preferably drebrin A knockout form.

The expression level of drebrin A and/or drebrin E, preferably both drebrin A and drebrin E, in the genetically recombinant non-human animal of the invention refers to 90% or less, 80% or less, 70% or less, 60% or less, or 55% or less, and 10% or greater, 20% or greater, 30% or greater, 40% or greater, or 45% or greater relative to that of the non-human animal Alzheimer's disease model used as a base. Specifically, the expression level of drebrin is preferably 10% to 90%, 20% to 80%, 30% to 70%, 40% to 60%, or 45% to 55% of that of the base non-human animal Alzheimer's disease model. In a preferred embodiment, drebrin genes are not completely knocked out in the genetically recombinant non-human animal model of the invention. A decrease in the expression level of drebrin described above is preferably a decrease in the expression level in the brain. This can be determined by a method in which brain homogenate is subjected to acrylamide gel electrophoresis, transferred onto a PVDF (polyvinylidene fluoride) membrane, then stained with a drebrin antibody, and the staining intensity of bands are compared.

Examples of methods of heterozygous knockout of a drebrin gene include a method of crossbreeding a base non-human animal Alzheimer's disease model with a drebrin gene knockout non-human animal. In this regard, either a heterozygote or a homozygote can be used as the drebrin gene knockout non-human animal, but it is preferable to use a homozygote because a step of selecting out a heterozygous knockout form from non-human animals born by crossbreeding would not be required. In the present invention, a drebrin gene knockout non-human animal may be crossbred once with a base non-human animal Alzheimer's disease model to prepare a genetically recombinant non-human animal model having a heterozygous Alzheimer's disease genetic mutation, or a drebrin gene knockout non-human animal may be crossbred twice with a base non-human animal Alzheimer's disease model to prepare a genetically recombinant non-human animal model having homozygous Alzheimer's disease genetic mutations. The genotype of an offspring obtained by crossbreeding a base non-human animal Alzheimer's disease model with a drebrin gene knockout non-human animal can be determined using a known genotyping method. For example, DNA sequencing, SSCP (Single Strand Conformation Polymorphism), RFLP (Restriction Fragment Length Polymorphism), PCR (Polymerase Chain Reaction), AFLP (Amplified Fragment Length Polymorphism), ASO (Allele Specific Oligonucleotide) probing method, a method of detecting attachment to a DNA microarray or DNA beads, or the like can be used. For example, the genetically recombinant non-human animal model of the invention obtained by such a method can be further crossbred with a base non-human animal Alzheimer's disease model and an offspring having a desired genotype can be selected by the genotyping method described above for passaging.

The drebrin gene knockout non-human animal described above can be prepared by, for example, deleting a region comprising exon 3 of a drebrin gene. While any known method can be used as a method of deleting a part of a drebrin gene, a Cre-loxP system derived from bacteriophage P1 in particular can bring in a mutation specifically inside the brain and can elicit impaired maturation of synapses (synaptic vulnerability), and therefore can be suitably used for the preparation of the genetically recombinant non-human animal of the invention. Instead of the Cre-loxP system described above, Saccharomyces cerevisiae derived FLP-FRT system, Zygosaccharomyces rouxii derived R-RS system, or bacteriophage Mu derived Gin-gix system can also be used.

The genetically recombinant non-human animal of the invention is useful in the analysis of the development mechanism of Alzheimer's disease. Furthermore, the genetically recombinant non-human animal of the invention can be used in screening of an agent for treating and/or preventing Alzheimer's disease. Examples of the method for screening as described above include a method comprising (a) administering a test substance to the genetically recombinant non-human animal of the invention; and (b) evaluating a therapeutic and/or prophylactic effect on Alzheimer's disease. Examples of the step of evaluating a therapeutic effect on Alzheimer's disease in (b) described above include a method of administering a test substance to the genetically recombinant non-human animal of the invention exhibiting a symptom of Alzheimer's disease to determine a therapeutic effect on Alzheimer's disease using, as an indicator, accumulation of amyloid β in the brain, the amount of PSD-95 protein, morphology or function of synapses, spatial memory or spatial working memory capability, coordinated movement, or motor learning. Examples of the step of evaluating a prophylactic effect on Alzheimer's disease in (b) described above include a method of administering a test substance to the genetically recombinant non-human animal of the invention prior to exhibiting a symptom of Alzheimer's disease to determine a prophylactic effect on the development of Alzheimer's disease using, as an indicator, accumulation of amyloid β in the brain, the amount of PSD-95 protein, morphology or function of synapses, spatial memory or spatial working memory capability, coordinated movement, or motor learning.

While the method of preparing a knockout mouse of the invention is specifically described hereinafter in the Examples, the technical scope of the invention is not limited to such exemplifications.

Example 1

1. Preparation of drebrin knockout (DXKO) mice

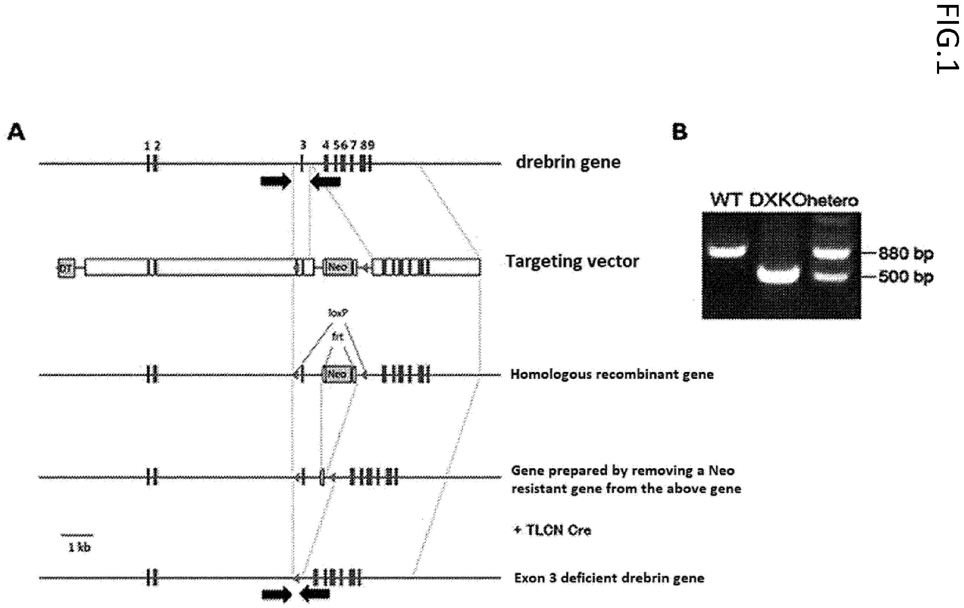

A DXKO mouse with a deletion of exon 3 (GGCTCTGTACACATACGAGGATGGCTCAGATGACCTCAAGCTTGCAGCGTCAGGAG; SEQ ID NO: 1) of a Dbn1 gene was prepared by crossbreeding a drebrin floxed mouse with a TLCN-Cre mouse (Fuse et al., Biochem BiophysRes Commun. 2004 Jun 4; 318(3): 665-72). The schematic diagram of the preparation method is shown in Figure 1A. The preparation method is specifically described hereinafter.

A drebrin gene of a C57BL/6J mouse was cloned by PCR. loxP was inserted 168 bases upstream of exon 3, a neomycin (neo) resistant gene (pKG-neo) flanked by frt sequences was inserted 318 bases downstream, and the other loxP was inserted 24 bases further downstream, and a diphtheria toxin A gene (DT) for negative selection was inserted at the 5' end to prepare a targeting vector. The targeting vector was introduced in ES cells (RENKA) established in the C57BL/6N mouse by electroporation, and ES clones that survived and grew in the presence of G418 were isolated. Isolated ES clones with a targeting vector that was correctly homologously recombined were sorted out by Southern blotting. From the resulting recombinant ES cells, neo resistant gene was removed. The ES cells were microinjected into the morulas derived from ICR mice, which were transplanted into the pseudopregnant foster parent's uterus to obtain chimeric mice. Drebrin floxed mice with a recombinant gene, which have a chimeric individual with a high ratio of somatic cells derived from ES cells among the resulting chimeric mice and a wild-type C57BL/6N mouse as parents, were obtained. Drebrin floxed mice were deposited with RIKEN BioResource Research Center, Experimental Animal Division (3-1-1 Takanodai, Tsukuba-shi, Ibaraki-ken, Japan) (Deposit number RBRC10925; C57BL/6N-Dbn1<tm2.1Shira>, DXKO-Flox) on September 27, 2019.

The resulting drebrin floxed mouse and a TLCN-Cre mouse were crossbred as parents to obtain heterozygous individuals of mice with a deletion of a region flanked by two loxP (DXKO heterozygous mice). The resulting DXKO heterozygous mice were crossbred to obtain DXKO homozygous individuals (DXKO homozygous mice). DXKO homozygous mice and DXKO heterozygous mice were sorted by genotyping through PCR (Figure 1B). As shown in Figure 1B, PCR amplification and electrophoresis of the region flanking exon 3 (region flanked by arrows in Figure **1A**) resulted in amplification of only a 500 bp fragment with a deletion of exon 3 in DXKO homozygous mice, and amplification of a 500 bp fragment with a deletion of exon 3 and a 880 bp fragment comprising exon 3 in DXKO heterozygous mice.

Example 2

2. Analysis of drebrin expression levels in DXKO mice

The cerebral cortex of each of wild-type (WT), DXKO heterozygous mouse (DXKO$^{+/-}$), and DXKO homozygous mouse (DXKO$^{-/-}$) was homogenized using SDS-sample buffer and heated, and then 100 μg and 50 μg of homogenates in wet weight were subjected to acrylamide gel electrophoresis. After transferring onto a PVDF membrane, a drebrin antibody (M2F6 hybridoma serum (Shirao and Obata, 1986; Non Patent Literature 9), RRID: AB_2532045) was used for detection. β-actin was used as a loading control, and detection was performed with an anti-actin antibody (Sigma-Aldrich A5441, Sigma-Aldrich).

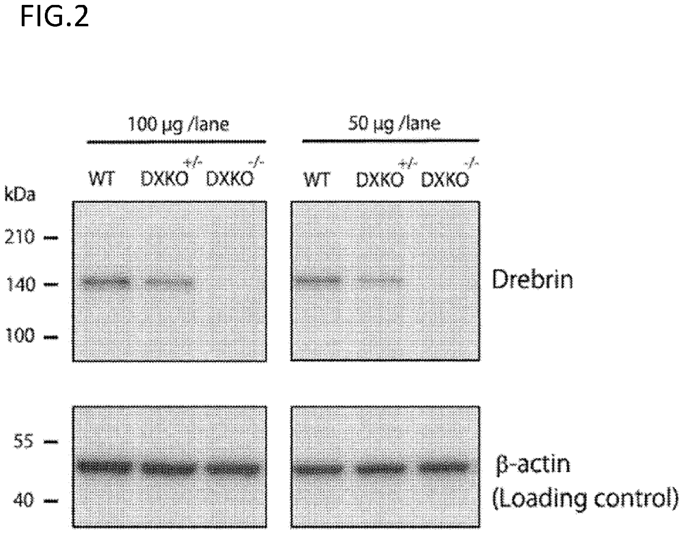

The results are shown in Figure **2**. While drebrin expression was not observed in DXKO homozygous mice, about half of drebrin expression of wild-type was detected in DXKO heterozygous mice.

Example 3

3. Observation of neurons in DXKO mice

Hippocampal neurons of wild-type (WT), DXKO heterozygous mouse (Het), and DXKO homozygous mouse (KO) were cultured on a 96-well plate, immobilized on day 21 of culture, and subjected to immunocytochemistry using a drebrin antibody (M2F6 hybridoma serum (Shirao and Obata, 1986; Non Patent Literature 9), RRID: AB_2532045) or an anti-MAP2 antibody (Sigma-Aldrich M4403, Sigma-Aldrich) and nuclear staining using DAPI, then images were captured using IN Cell Analyzer 2200 (GE Healthcare). The viable neuron count, dendrite length, and drebrin aggregation image count (total drebrin cluster count and drebrin cluster density) were automatically analyzed by the method of Hanamura et al. (Hanamura et al., J Pharmacol Toxicol Methods. 2019 Jul 2: 106607. doi: 10.1016/j.vascn.2019.106607) by using IN Cell Developer Toolbox v1.9 (GE Healthcare).

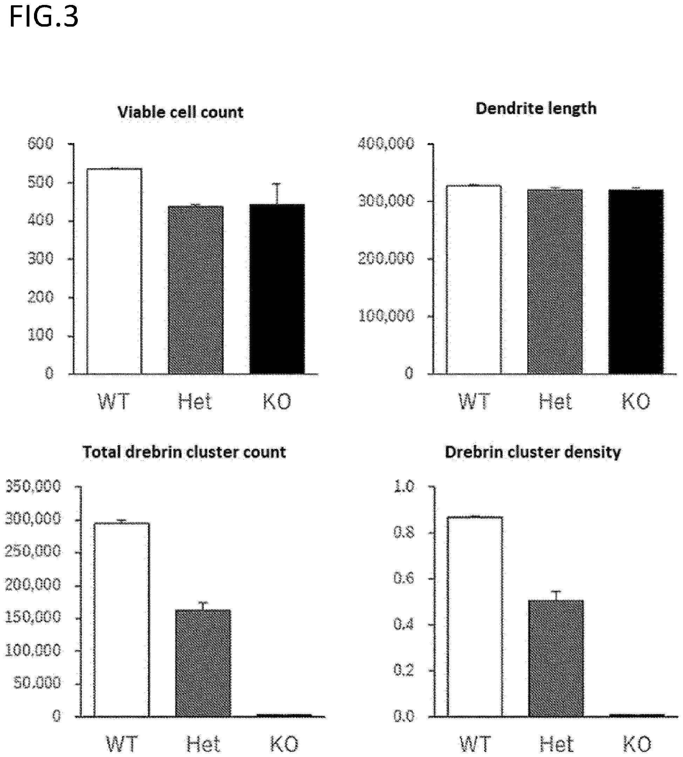

The results are shown in Figure **3.** While the viability rate and dendrite growth rate of neurons of wild-type (WT), DXKO heterozygous mouse (Het), and DXKO homozygous mouse (KO) did not change, the drebrin aggregation image count was about half in the DXKO heterozygous mouse and zero in the DXKO homozygous mouse with complete knockout of drebrin.

Example 4

4. Analysis of NMDA receptor activity

Frozen mouse hippocampal neurons of wild-type and DXKO heterozygous mice (hetero-type) were cultured for 3 weeks using a 96-well plate and then immobilized after treatment with 50 $\mu$M glutamic acid for 10 minutes. The drebrin cluster count was detected with a drebrin antibody (M2F6 hybridoma serum (Shirao and Obata, 1986; Non Patent Literature 9), RRID: AB_2532045). Pictures were captured and analyzed using IN Cell Analyzer 2200 (GE Healthcare) in accordance with the methods of Hanamura et al. (Hanamura et al., J Pharmacol Toxicol Methods. 2019 Jul 2: 106607. doi: 10.1016/j.vascn.2019.106607) and Mitsuoka et al. (J Pharmacol Toxicol Methods. 2019 May 10: 106583. doi: 10.1016/j.vascn.2019.106583).

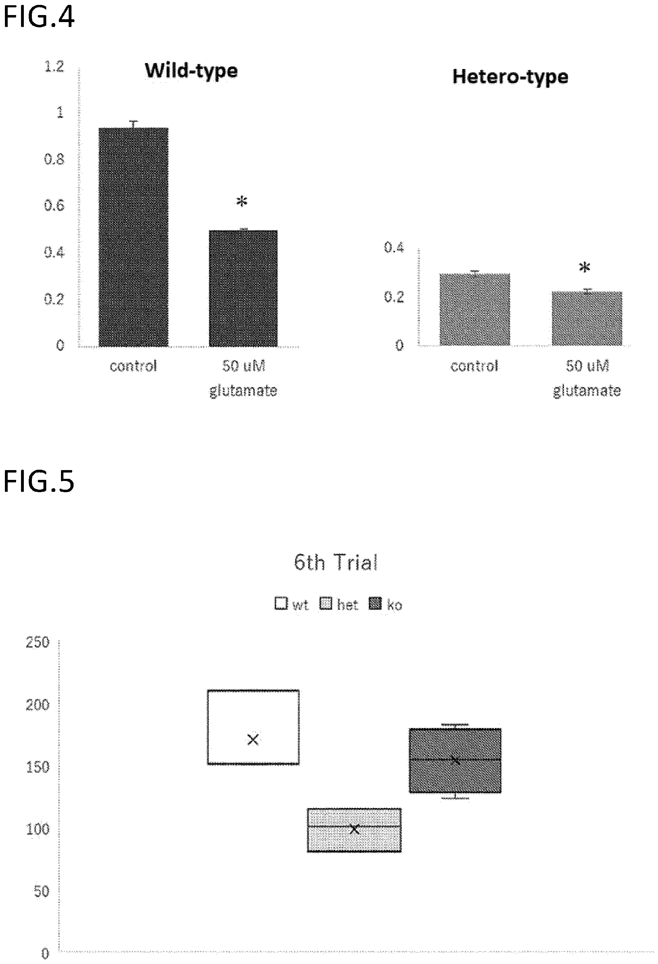

The results are shown in Figure **4.** NMDA receptor activity was maintained in wild-type and hetero-type, but further attenuation in activity was observed in hetero-type compared to wild-type.

Example 5

5. Analysis of dendritic spine stability

5-1 Comparison of wild-type and DXKO homozygous mice

In order to compare the spine stability of wild-type (WT) and DXKO homozygous (Homo) mice, Thin skull technique for thinning the skull under deep sedation through a mixture of three types of anesthetics to create an observation window was applied to mice (35 to 57 weeks old) prepared by crossbreeding with a transgenic mouse expressing YFP downstream of a Thy-1 promotor, and layer I dendrite spine among apical dendrites extending primarily from layer V pyramidal neurons of the cerebral neocortical somatosensory area was observed using a multiphoton excitation laser scanning microscope (FVMPE-RS, Olympus). These were basically performed based on the method of Takatsuru et al. (BrainRes. 2009 Oct 19; 1294: 45-51). After the first observation, the spine was observed again after 6 hours to study the new formation and elimination of spine that occurred during 6 hours. This suggested that there is no difference in the stability of spine (Table 1).

[Table 1]

| After 6 hours | New formation of spine | Elimination of spine |
|---|---|---|
| WT | 2.70% | 4.30% |
| Homo | 2.70% | 4.80% |

5-2 Comparison of wild-type and DXKO heterozygous mice

In order to compare the spine stability of wild-type (WT) and DXKO heterozygous (Hetero) mice, the mice were crossbred with a transgenic mouse expressing GFP downstream of a Thy-1 promotor, and Thin skull technique was applied under deep sedation through a ketamine/xylazine mixture, and layer I dendrite spine among apical dendrites extending primarily from layer V pyramidal neurons of the cerebral neocortical somatosensory area was observed using a multiphoton excitation laser scanning microscope. These were also performed based on the method of Takatsuru et al. described above. The new formation and elimination of spine that occurred during one week were studied for these mice (9 to 17 weeks old) to find that this occurred at a high rate in DXKO heterozygous mice, suggesting that the spine is more unstable in DXKO heterozygous mice than wild-type mice.

[Table 2]

| After 24 hours | New formation of spine | Elimination of spine |
| --- | --- | --- |
| WT | 2.40% | 2.40% |
| Hetero | 5.20% | 9.40% |

Example 6

6. Rotarod test

Wild-type (WT), DXKO heterozygous mouse (Het), and DXKO homozygous mouse (KO) (8 months old) were placed on a wheel with a 32 mm diameter that rotates with a starting rotational speed of 4 rpm (0 seconds) which gradually accelerates to 40 rpm (300 seconds) to measure the time until the mice fell off. After 5 trials of training, the time until falling on the sixth trial was used as the measurement result. The mean value was computed from three trials a day for 2 consecutive days.

The results are shown in Figure **5.** The DXKO heterozygous mice fell off earlier relative to wild-type and DXKO homozygous mice, suggesting that wild-type and homo-type mice exhibit no abnormality in coordinated movement or motor learning, but DXKO heterozygous mice do.

Example 7

7. Analysis of behavioral change

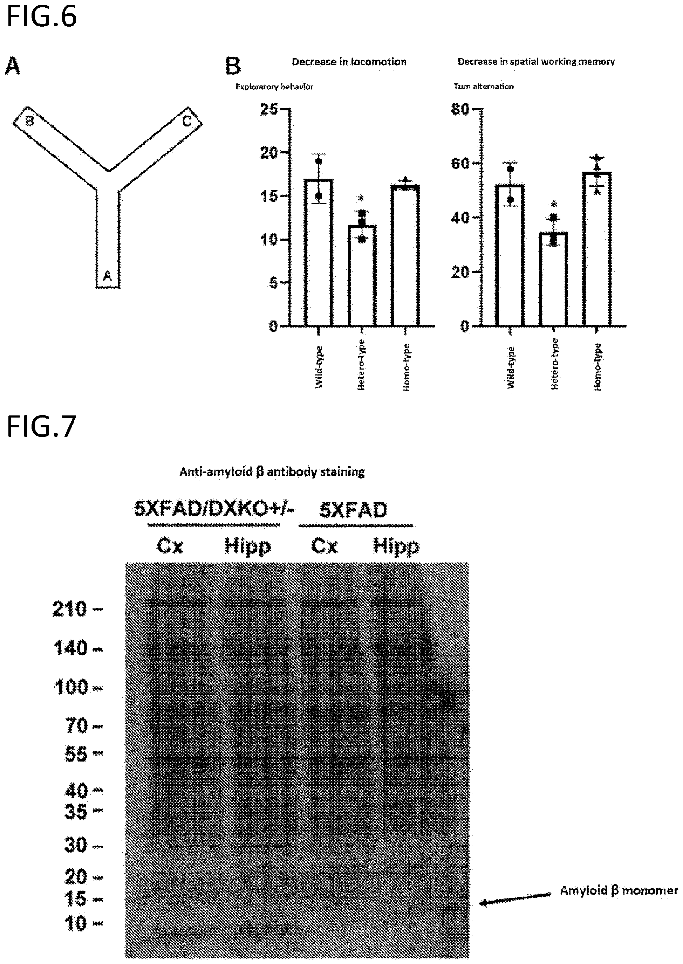

The Y-maze shown in Figure **6A** was used to analyze the behavioral change in wild-type (WT), DXKO heterozygous mouse (hetero-type), and DXKO homozygous mouse (homo-type) (8 months old). The Y-maze consists of a gray plastic center platform and three arms, where each arm has a dimension of 3 cm (bottom)/12 cm (top) width $\times$ 13 cm height $\times$ 40 cm length. The mice were placed on the center platform of the Y-maze and allowed to freely explore for 5 minutes. The number of times the mice enter an arm was used as an indicator of locomotion, and the voluntary turn alternation ratio calculated by the following equation (1) was used as the indicator of spatial working memory.

```
Voluntary turn alternation ratio (%) = [(voluntary turn
alternation)/(total number of entries - 2)] × 100 ................ (1)
```

*voluntary turn alternation: number of three consecutive entries into different arms

The results are shown in Figure **6B.** A decrease in locomotion and spatial working memory was not observed in homo-type relative to wild-type, but a decrease in both locomotion and spatial working memory was observed in hetero-type.

In view of the results of Examples 3 to 7, an abnormality in the viability rate of neurons or dendrite growth rate was not observed (Example 3), but the NMDA receptor function is diminished (Example 4), stability of the spine is decreased (high ratio of new formation and elimination) (Example 5), an abnormality is manifested in coordinated movement and motor learning (Example 6), and the spatial memory and spatial working memory are reduced (Example 7) in DXKO heterozygous mice. The reason why such an abnormality is observed only in heterozygous mice is conjectured to be because some type of compensatory function takes effect in DXKO homozygous mice that are completely devoid of drebrin congenitally, so that an abnormality found in heterozygous mice is not observed.

It is known that drebrin decreases with age. Thus, the abnormality in DXKO heterozygous mice described above possibly exhibited a phenotype reflecting senescence due to the drebrin expression level in DXKO heterozygous mice halving as seen in elderlies who are 60 years old or older. In this regard, the following experiment was conducted with an idea that an animal model having the strongest risk factor of Alzheimer's disease, i.e., senescence, can be created by halving the expression level from heterozygous knockout of a drebrin gene in conventional mouse Alzheimer's disease model.

Example 8

8. Preparation of Alzheimer's disease animal model with heterozygous knockout of drebrin gene (5xFAD/DXKO+/-)

Mice (5xFAD/DXKO+/-) heterozygously having a 5 x FAD gene and a drebrin gene (+/-) was prepared, as a mouse Alzheimer's disease model, by crossbreeding a 5xFAD mouse (MMRRC034848, The Jackson Laboratory) with a DXKO homozygous mouse. In this regard, a 5xFAD mouse is a mouse in which human APP695 cDNA and human PS1 cDNA with mutations in Swedish (K670N and M671L), Florida (I716V), and London (V717I) (mutations of M146L and L286V) are overexpressed under regulation of transcription of a neuron-specific mouse Thy-1 promotor, and accumulation of amyloid β proteins in the brain is promoted (Oakley, H. et al., J Neurosci, 26, 10129-10140, 2006).

Example 9

9. Amyloid β accumulation in the brain of 5xFAD/DXKO$^{+/-}$ mice

The amyloid β accumulation in the brain of the 5xFAD/DXKO$^{+/-}$ mice prepared in Example 8 and 5xFAD mice was compared. 500 μg of homogenates of the cerebral cortex (Cx) and hippocampus (Hipp) in wet weight extracted from the 5xFAD/DXKO$^{+/-}$ mice and 5xFAD mice (15 weeks old) were subjected to electrophoresis and Western blotting using an anti-amyloid β antibody (BioLegend).

The results are shown in Figure **7.** Comparison of bands of amyloid β monomers shows that the amyloid β monomer band is darker and amyloid β is accumulated more for 5xFAD/DXKO$^{+/-}$mice than 5xFAD mice in both the cerebral cortex and hippocampus.

Example 10

10. Comparison of PSD95 expression levels of 5xFAD/DXKO$^{+/-}$mice

PSD95 (postsynaptic density protein 95) is a key scaffolding protein at the postsynapse and is one of the proteins that is most abundant at the postsynaptic density (PSD). PSD95 is understood to contribute to the maintenance of synaptic function, plasticity, etc. by interacting with various molecules such as NR2A-D, GluR6, neuroligin, and nNOS.

In this regard, the PSD95 expression levels of the 5xFAD/DXKO$^{+/-}$ mice prepared in Example 8 and 5xFAD mice were compared. 300 μg of homogenates of the cerebral cortex (Cx) and hippocampus (Hipp) in wet weight extracted from the 5xFAD/DXKO$^{+/-}$ mice and 5xFAD mice (15 weeks old) were subjected to electrophoresis and Western blotting using an anti-PSD95 antibody (Thermo Scientific).

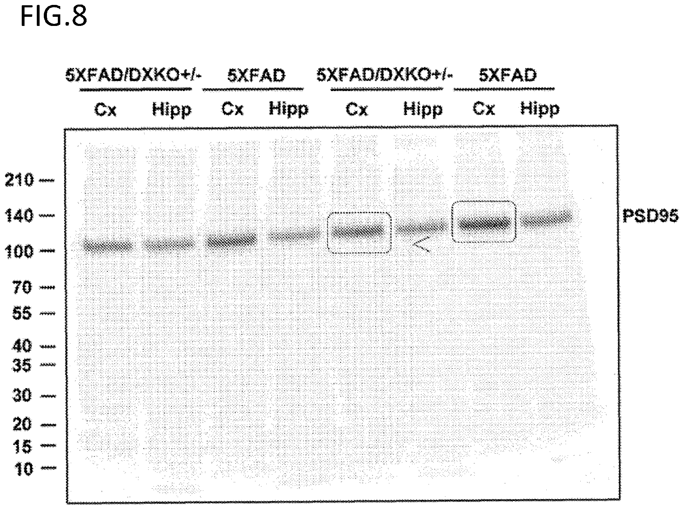

The results are shown in Figure **8.** Although a difference in intensity of bands of PSD95 for the 5xFAD/DXKO$^{+/-}$ mice and 5xFAD mice was not observed in the hippocampus, bands of amyloid β monomer was lighter for the 5xFAD/DXKO$^{+/-}$ mice than the 5xFAD mice. This shows that expression of PSD95 is reduced in 5xFAD/DXKO$^{+/-}$ mice.

In view of the results of Examples 9 and 10, amyloid β accumulation is promoted, and expression of PSD95, which is understood to contribute to maintenance of normal synaptic function and plasticity, is reduced in 5xFAD/DXKO$^{+/-}$ mice relative to 5xFAD mice. This suggests that mouse Alzheimer's disease models having heterozygous knockout of a drebrin gene to halve drebrin expression reflect the pathological condition of Alzheimer's disease more accurately.

[Industrial Applicability]

The present invention can readily achieve synaptic vulnerability due to senescence, which is difficult to achieve in short-lived animal models such as mice, by halving drebrin. A non-human animal Alzheimer's disease model with a risk of senescence can be created by crossbreeding a conventional non-human animal Alzheimer's disease model that requires senescence with a drebrin knockout non-human animal to produce a drebrin heterozygous knockout non-human animal.

Animal models (AD animals) prepared by the present invention have pathological findings of Alzheimer's disease such as promotion of amyloid plaque deposition as well as the strongest risk factor of Alzheimer's disease, i.e., senescence. Thus, the animal models can be an excellent animal model for human Alzheimer's disease that was not available in the past, and can be used in drug development for dementia such as Alzheimer's disease.

Since cultured neurons produced from animal models prepared by the present invention have drebrin accumulation in the synapses reduced to about 50% just like elderly humans, the animal model can be used as a model for neurons in patients of Alzheimer's disease developed in elderly patients and can be used in drug development for dementia such as Alzheimer's disease.

Therefore, industrial applicability of the present invention is very high in the medical and pharmaceutical fields.

**Claims**

1. A genetically recombinant non-human animal **characterized by** heterozygous knockout of a drebrin gene of a base non-human animal Alzheimer's disease model.

2. The genetically recombinant non-human animal of claim 1, **characterized by** being a drebrin A and drebrin E heterozygous knockout form.

3. The genetically recombinant non-human animal of claim 1 or 2, **characterized in that** the base non-human animal Alzheimer's disease model is a non-human animal Alzheimer's disease model with accumulation of amyloid β in a brain.

4. The genetically recombinant non-human animal of any one of claims 1 to 3, **characterized in that** the base non-human animal Alzheimer's disease model is a mouse Alzheimer's disease model.

5. The genetically recombinant non-human animal of claim 4, **characterized in that** the mouse Alzheimer's disease model is a 5 x FAD mouse.

6. A method of preparing a genetically recombinant non-human animal that develops Alzheimer's disease, **characterized in that** a base non-human animal Alzheimer's disease model is crossbred with a drebrin gene knockout non-human animal.

7. A method of screening an agent for treating and/or preventing Alzheimer's disease, comprising the following steps (a) and (b):

   (a) administering a test substance to the genetically recombinant non-human animal of any one of claims 1 to 5; and
   (b) evaluating a therapeutic and/or prophylactic effect on Alzheimer's disease.

# FIG.1

# FIG.2

# FIG.3

# FIG.4

# FIG.5

# FIG.6

**A**

**B**

Decrease in locomotion

Exploratory behavior

Decrease in spatial working memory

Turn alternation

# FIG.7

Anti-amyloid β antibody staining

FIG.8

## INTERNATIONAL SEARCH REPORT

International application No.

PCT/JP2020/038004

**A. CLASSIFICATION OF SUBJECT MATTER**
C12N 15/12(2006.01)i; G01N 33/15(2006.01)i; A01K 67/027(2006.01)i
FI: A01K67/027 ZNA; C12N15/12; G01N33/15 Z
According to International Patent Classification (IPC) or to both national classification and IPC

**B. FIELDS SEARCHED**

Minimum documentation searched (classification system followed by classification symbols)
C12N15/12; G01N33/15; A01K67/027

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS
(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | LIU, Y. et al., "Down-Regulated Drebrin Aggravates Cognitive Impairments in a Mouse Model of Alzheimer's Disease", International Journal of Molecular Sciences, 2017, vol. 18, no. 4, pp. 1-17, abstract, fig. 2-4, 6 | 1-4, 6-7<br>5, 7 |
| Y | LEE, J. E. et al., "An Update of Animal Models of Alzheimer Disease with a Reevaluation of Plaque Depositions", Experimental Neurobiology, 2013, vol. 22, no. 2, pp. 84-95, abstract | 5, 7 |

☐ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 01 December 2020 (01.12.2020) | 15 December 2020 (15.12.2020) |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
|---|---|

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **TAKASHI SAITO ; TAKAOMI NISHIMICHI.** Arutsu-haimabyo no Moderu Mausu [mouse model for Alzheimer's disease. *Folia Pharmacol. Jpn.,* 2014, vol. 144, 250-252
- *Aging Cell,* 2018, vol. 17, e12802
- *J. Neurochem.,* 1985, vol. 44, 1210-1216
- *J. Biochem.,* 1995, vol. 117, 231-236
- *J. Neurosci. Res.,* 1994, vol. 38, 149-159
- *Cell Res.,* 1994, vol. 215, 145-153
- *J. Biol. Chem.,* 1994, vol. 269, 29928-29933
- *J. Neurosci.,* 1996, vol. 15, 7161-7170
- *Dev. Brain Res.,* 1986, vol. 29, 233-244
- *Brain Res.,* 1987, vol. 413, 374-378
- *J Neurosci. Res.,* 1996, vol. 43 (1), 87-92
- *Neuroscience.,* 2010, vol. 165 (1), 138-50
- *Eur. J. Neuroscience,* 2017, vol. 46, 2214-2228
- *J Neuropathol Exp Neurol.,* June 1999, vol. 58 (6), 637-43
- **FUSE et al.** *Biochem BiophysRes Commun.,* 04 June 2004, vol. 318 (3), 665-72
- **HANAMURA et al.** *J Pharmacol Toxicol Methods.,* 02 July 2019
- **MITSUOKA et al.** *J Pharmacol Toxicol Methods.,* 10 May 2019
- **TAKATSURU et al.** *BrainRes.,* 19 October 2009, vol. 1294, 45-51
- **OAKLEY, H. et al.** *J Neurosci,* 2006, vol. 26, 10129-10140

SEQUENCE LISTING

```
<110>  AlzMed, Inc.

<120>  NONHUMAN MODEL ANIMAL OF ALZHEIMER'S DISEASE AND METHOD FOR
MANUFACTURING THE SAME

<130>  2020C13074

<150>  JP 2019-185245
<151>  2019-10-08

<160>  1

<170>  PatentIn version 3.1

<210>  1
<211>  56
<212>  DNA
<213>  Mus musculus

<220>
<221>  misc_feature
<223>  Inventor:SHIRAO, Tomoaki; SEKINO, Yuko; HANAMURA, Kenji; YAMAZAKI
       , Hiroyuki; KOGANEZAWA, Noriko


<400>  1
ggctctgtac acatacgagg atggctcaga tgacctcaag cttgcagcgt caggag            56
```